(19) Europäisches Patentamt
European Patent Office
Office européen des brevets

(11) **EP 1 703 933 B1**

(12) **EUROPEAN PATENT SPECIFICATION**

(45) Date of publication and mention
of the grant of the patent:
**27.07.2016 Bulletin 2016/30**

(21) Application number: **05705080.9**

(22) Date of filing: **06.01.2005**

(51) Int Cl.:
***A61M 16/00*** (2006.01)

(86) International application number:
**PCT/US2005/000281**

(87) International publication number:
**WO 2005/070488 (04.08.2005 Gazette 2005/31)**

(54) **SENSORLESS PROPORTIONAL POSITIVE AIRWAY PRESSURE APPARATUS**

SENSORLOSES PROPORTIONALES POSITIVES ATEMDRUCKGERÄT

APPAREIL DE PRESSION POSITIVE EXPIRATOIRE PROPORTIONNELLE EXEMPT DE DETECTEUR

(84) Designated Contracting States:
**DE ES FR GB IT**

(30) Priority: **09.01.2004 US 535379 P**

(43) Date of publication of application:
**27.09.2006 Bulletin 2006/39**

(73) Proprietor: **SENSORMEDICS CORPORATION**
**Yorba Linda CA 92887 (US)**

(72) Inventor: **HIGGINS, John**
**Santa Ana, California 92704 (US)**

(74) Representative: **Viering, Jentschura & Partner**
**mbB**
**Patent- und Rechtsanwälte**
**Grillparzerstraße 14**
**81675 München (DE)**

(56) References cited:
**WO-A-01/19439       WO-A-02/32489**
**WO-A1-97/10019    US-A1- 2002 078 958**
**US-B1- 6 609 517**

- **FARRE ET AL: "Flow-dependent positive airway pressure to maintain airway patency in sleep apnea-hypopnea syndrome." AMERICAN JOURNAL OF RESPIRATORY AND CRITICAL CARE MEDICINE. JUN 1998, vol. 157, no. 6 Pt 1, June 1998 (1998-06), pages 1855-1863, XP002328111 ISSN: 1073-449X**

## Description

The present invention generally relates to pressurized delivery devices. Specifically, the present invention relates to positive airway pressure devices for treating sleep-related breathing disorders.

BACKGROUND OF THE INVENTION

Sleep-related breathing disorders, such as obstructive sleep apnea syndrome (OSAS) and chronic obstructive pulmonary disorder (COPD), adversely affect breathing during sleep. Breathing disruption often results from the collapse or closing of an individual's air passageway. OSAS, as one common example of a sleep-related breathing disorder, is an abnormal physical condition that affects a person's ability to breathe properly after falling asleep. Persons suffering from sleep apnea can stop breathing for periods as short as a few seconds and as long as a few minutes.

Typically, sleep-related breathing disorders such as OSAS are treated by Continuous Positive Air Pressure (CPAP) therapy. In CPAP therapy, a device forces air into an individual's air passageway. The CPAP device maintains sufficient pressure to keep the air passageway open during periods of sleep. The patient wears a mask-like device that is connected to the CPAP device to provide an elevated air pressure in the patient's upper air passageway. Traditional CPAP therapy ignores variations in pressure requirements and provides therapy at one pressure level and subjects the patient to the same positive airway pressure during exhalation as inspiration. For patients that require high therapeutic pressures, the high exhalation pressure causes patient discomfort and may compromise patient compliance. CPAP therapies therefore operate based on a worst-case treatment parameter, which is peak pressure requirements during inspiration.

Other therapies known as BiPAP (Bi-Level Positive Airway Pressure) operate at two positive air pressure levels. A lower pressure level is used during exhalation while a higher pressure level is used during inhalation.

The BiPAP device makes it easier to exhale due to the lower pressure used during exhalation. BiPAP devices are often prescribed for individuals that require higher pressures than those present in CPAP devices. BiPAP devices reduce the exhalation pressure by an amount proportional to the exhaled flow of the patient, without flow sensing. The cost and reliability burdens associated with flow sensing are eliminated. Bi-level therapies seek to take advantage of the different pressure requirements to lower the pressure during exhalation. Nevertheless, bi-level therapies also do not afford optimal treatment because the inspiratory positive airway pressure (IPAP) of bi-level PAP is again based on the patient's peak needs encountered during inspiration and remains constant over the entire inspiratory phase respiration. Also, during bi-level treatment, the expiratory position airway pressure (EPAP) remains constant and is related solely to the support needs at the end of exhalation.

WO 97/10019 A1 discloses a positive airway pressure apparatus comprising a flow generator coupled to the a delivery device, the flow generator producing a pressurized gas to the delivery device; a controller having a control signal input and a summing component and configured to produce an output signal that drives the flow generator; and a pressure sensor configured to sense pressure in the positive airway pressure apparatus, the pressure sensor providing a pressure sensor input signal representing the pressure to the controller.

SUMMARY OF THE INVENTION

The present invention provides a positive airway pressure apparatus in which the pressure output is modulated in proportion to the patient's breathing pattern to reduce the work of breathing in the patient while maintaining the required prescribed pressure during inspiration. This is performed with a pressure regulator design whose intrinsic behavior is such that it presents to the patient a controlled negative pressure impedance without the need for any flow sensing device outside the basic pressure regulator.

The invention provides an apparatus according to claim 1.

Further embodiments of the invention are described in the dependent claims.

BRIEF DESCRIPTION OF THE DRAWINGS

   FIG. 1 is a block diagram illustration of the positive airway pressure apparatus of the present invention;
   FIG. 2 is a graphical representation of a typical breathing pattern of a patient;
   FIG. 3 is a graphical representation of one controller mode for a positive airway pressure apparatus of the present invention;
   FIG. 4 is a graphical representation of another controller mode for a positive airway pressure apparatus of the present invention; and

FIG. 5 is a graphical representation of a frequency spectrum of a leak.

DETAILED DESCRIPTION OF THE EMBODIMENTS

In the following description of the present invention reference is made to the accompanying drawings which form a part thereof, and in which is shown, by way of illustration, exemplary embodiments illustrating the principles of the present invention and how it may be practiced. It is to be understood that other embodiments may be utilized to practice the present invention and structural and functional changes may be made thereto without departing from the scope of the present invention.

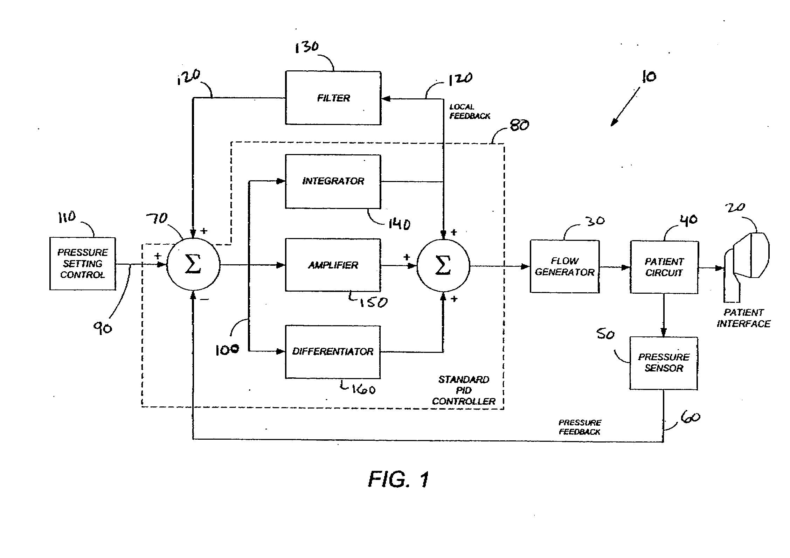

FIG. 1 shows a block diagram representation of a positive airway pressure apparatus 10. Apparatus 10 delivers pressurized breathing gas to a patient via patient interface 20 in proportion to the patient's respiratory flow for treatment of sleep-related breathing disorders such as OSAS, COPD and other conditions. Apparatus 10 includes components that deliver pressure proportional to the patient's respiratory flow without sensing gas flow.

Apparatus 10 includes a gas flow generator 30, such as a conventional CPAP blower, that receives gas from any suitable source. The pressurized gas is delivered to the patient via patient circuit 40 and patient interface 20.

The apparatus also includes a pressure sensor 50 that senses gas pressure. Pressure sensor 50 may sense gas pressure anywhere between and including the output of flow generator 30 and patient interface 20. Pressure sensor 50 may also sense gas pressure from within the flow generator 30 itself. The pressure sensor 50 produces a pressure signal 60 (also referred to herein as pressure sensor input signal or pressure feedback input) that represents gas pressure in the apparatus 10 which is fed back to a summing junction 70 in a controller 80. The summing junction 70 subtracts the pressure signal 60 from a control signal 90 (also referred to herein as control signal input or pressure control input or control signal input) to produce an error signal 100 that is processed by the various components of the controller 80. The output of the controller 80 varies the power output of the flow generator 30 such that the pressure signal 60 converges to the control signal 90 that is set by a pressure setting control 110. The components of the controller 80 are configured in such a way that unconditional and controlled convergence is insured. The output of pressure setting control 110 (which is control signal 90) is not necessarily fixed and may vary over time according to various titration features incorporated into the apparatus 10. Under all operational conditions, the pressure signal 60 will track the control signal 90.

The apparatus 10 of the present invention also includes a feedback system comprised of a feedback signal 120 (also referred to as local feedback input) which follows feedback path 120. The source for the feedback signal 120 may be any point between the output of summing junction 70 and the input to flow generator 30. In the embodiment of FIG. 1, the sou rce for feedback signal 120 is the output of an integrator 140, which is one example of an internal component of the controller 80. In another embodiment, feedback signal 120 is modified by a filter 130 and added to control signal 90 and pressure signal 60 at summing junction 70. Filter 130 may include a band-pass filter, a signal compressor, and a rectifier. However, filter 130 is not limited to this composition, and can be constituted in any number of ways to tailor the behavior of controller 80.

FIG. 1 shows that in one embodiment, the controller 80 may include components such as an integrator 140, an amplifier 150, and a differentiator 160. Controller 80 may also be a standard PID controller. In this specification, the description of the controller 80 is included for illustrative purposes only. It is to be understood that controller 80 may include additional or different components and that controller configurations other than that shown in FIG. 1 can be utilized for this invention. Additionally, controller 80 represents an example of a feedback control implementation that is widely known in the art. It is to be understood that any form of feedback control implementation can be utilized for this invention.

The introduction of the feedback system or loop and the feedback signal 120 provides a pressure regulator design whose intrinsic behavior is such that it presents to the patient a controlled *negative* pressure impedance without the need for any sensing device outside the basic pressure regulator. Therefore, the present invention does not use a sensor to sense any other attributes of the substance delivered, such as flow. The present invention only includes a pressure sensor 50 for sensing the pressure in the positive airway pressure apparatus 10.

To elucidate the concept of negative pressure impedance, first consider a simple unregulated pressure delivery device. This type of device presents a *positive* pressure impedance to the patient. That is, pressure increases if the patient exhales and decreases when the patient inhales. Now, consider a device that regulates pressure. Here, pressure remains constant regardless of respiratory flow. Hence, this device presents a zero pressure impedance to the patient. With the present invention, the mask pressure *decreases* as the patient exhales and *increases* as the patient inspires, presenting a *negative* pressure impedance to the patient. This behavior has the desired effect of reducing breathing effort while maintaining prescribed inspiration pressure.

To create negative pressure impedance with a typical pressure-regulated CPAP device, an additional localized feedback path is added to the controller 80. A component of the controller's internal error signal is fed back to a third non-inverting input of the controller 80 such that the feedback is positive in polarity.

FIG. 1 shows that in one embodiment of the present invention, the polarity of feedback path 120 is positive. A positive going signal from the output of the integrator 140 produces a positive going signal at its input (e.g., via the feedback path 120 and summing junction 70). Positive feedback along this path 120 modifies the behavior of controller 80 such that its regulated pressure output has a negative pressure impedance. This can be understood by examining what happens when a patient exhales into patient interface 20. In normal CPAP mode (i.e., no positive feedback), the expiration flow will cause the pressure in patient circuit 40 to increase, resulting in an increase of the pressure signal 60. Pressure signal 60 and control signal 90 will no longer match and their sum (i.e., error signal 100) will become negative. This negative going signal is amplified by the components of the controller 80, causing its output to go more negative which in turn slows down flow generator 30, causing the pressure to drop in compensation for the initial increase. Eventually (on the order of milliseconds), the pressure will drop to the point where the output of summing junction 70 returns to zero. In short, the control loop always operates in a direction that forces the output of the summing junction 70 towards zero, which is the point that the pressure signal 60 matches the control signal 90.

Under this same scenario, with positive feedback added, the negative going output of the controller 80 also causes a third non-inverting input to the summing junction 70 to become negative. This causes the output of summing junction 70 to become zero at a point where the pressure signal 60 is *less* than the control signal 90. As long as the patient continues to exhale, the controller 80 will regulate at a pressure lower than that commanded by control signal 90. The opposite process occurs when the patient inspires, unless the feedback signal 120 is blocked during this cycle (i.e., rectification). In the opposite process, as long as the patient continues to inhale, the output of the summing junction 70 will become zero at a point where the pressure signal 60 is greater than the control signal 90, and the controller 80 will regulate at a pressure higher than that commanded by control signal 90. This process is continuous and roughly proportional to respiratory flows in a stable system. The magnitude of this pressure response to respiratory flow is controlled by the gain of filter 130. This gain does not need to be linear. In fact, the gain might be compressed somewhat to suppress excessive responses to sighs and coughs.

In one embodiment, this feedback signal is band-limited to the frequencies necessary to resolve normal respiratory activity. Negative resistance response to flows related to leaks, for example, will be blocked by removing the leak's frequency spectrum from the feedback signal. Response to sighs and coughs can be attenuated in a similar fashion.

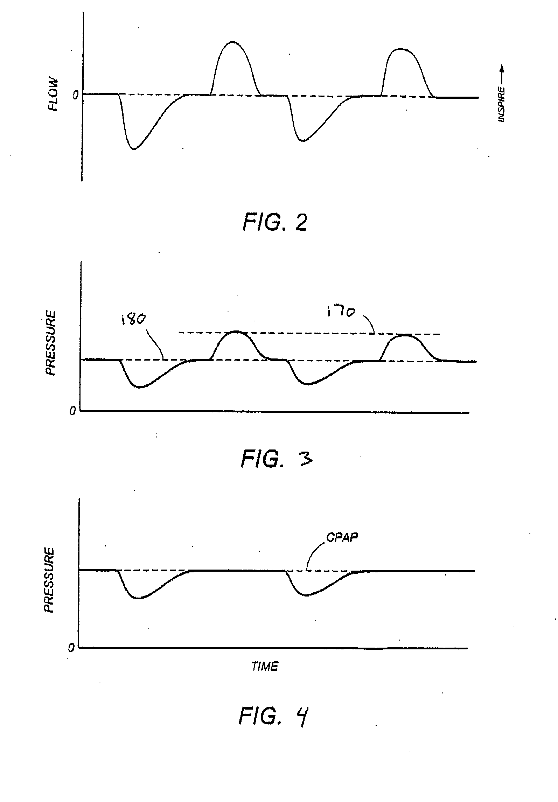

Conditioning of the feedback signal 120 is not limited to band-pass filtering nor need it be linear. The feedback signal 120 can be modified in any number of ways to achieve some desired behavior of the controller 80. For example, it can be compressed to improve the controller's immunity to sighs or coughs. It can also be rectified to cause the controller 80 to operate in "half-wave" mode. In "half-wave" mode, shown graphically in FIG. 4, negative pressure impedance is presented only during the expiration cycle of a breath, leaving the pressure at a preset value during inspiration. This reduces the pressure during exhalation while leaving the inspiration pressure at a normal CPAP level. The "full-wave" mode, shown graphically in FIG. 3, presents negative pressure impedance over the entire breathing cycle. This mode can optimally minimize the patient's breathing effort while maintaining sufficient support pressure during inspiration.

Controller 80 represents an example of a feedback control implementation that is widely known in the art. It is to be understood that any form of feedback control implementation can be utilized for this invention.

The pressure signal 60 from the pressure sensor 50 may have a negative polarity. Because implementation of a positive polarity feedback path can sometimes result in an unstable controller, the controller 80 will be stable if the negative feedback path magnitude exceeds the positive feedback path magnitude minus one, as defined by Eq. (1):

$$\left| Feedback_{negative} \right| > \left| Feedback_{positive} \right| -1 = \text{TRUE}$$

$$(1)$$

Satisfying Eq. (1) will result in a stable system. With the gain of filter 130 set appropriately, and with a properly designed band-pass filter, the apparatus 10 allows large pressure responses to respiratory flows while satisfying Eq. (1). In theory, no fundamental limit exists for the maximum attainable stable pressure responses. In practice, pressure response magnitudes on the order of 12 cm $H_2O$ $L^{-1}$ $sec^{-1}$ have been easily achieved while maintaining stability.

Filter 130 is included in the apparatus 10 to limit the controller's negative pressure impedance response to the patient's normal breathing pattern. Response to flow events such as leaks, sighs or coughs are either attenuated or eliminated by inclusion of filter 130. In addition to this, filter 130 can be configured to remove the controller's response to inspiratory flows for simpler systems that lower pressure only during exhalations.

FIG. 2 is a graphical representation of a patient's typical breathing pattern, showing normal flow during inspiration and expiration. FIG. 3 is a graphical representation of a response to a patient's breathing pattern. Reference numeral 170 shows a conventional prior art CPAP response, in which a constant high pressure is delivered during both inspiration and expiration. Reference numeral 180 shows a typical response as in the present invention, in which a baseline pressure provides sufficient pressure during inspiration and expiration that improves patient comfort and compliance. FIG. 3 therefore displays a "full-wave" response to both inspiration and expiration. FIG. 4 is a graphical representation of a "half-wave" response showing a baseline pressure delivered only during expiration. FIG. 4 shows a response as discussed below in an embodiment in which a recitifier is included with filter 130.

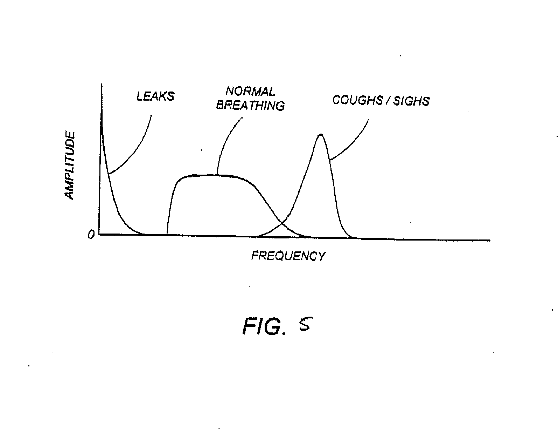

FIG. 5 is a graphical representation of a frequency spectrum of a leak. In one embodiment, filter 130 includes a band-pass filter configured to discriminate between normal breathing patterns and all other flow events. As shown in FIG. 5, the frequency spectrum of a leak (in terms of flow modulation) is weighted heavily toward low frequencies below typical breath rates and is easily discriminated. The high-pass component of the band-pass filter in filter 130 can be designed with a sharp cutoff just below the lowest anticipated breath rate to block these frequencies. The DC component of a leak, which is most significant, will be completely blocked by this type of filter. This frequency discrimination can take on many forms depending on the application and type of patient interface 20 (nasal vs. mask, for example).

Sighs and coughs can be treated in a similar fashion. They are weighted toward frequencies higher than the breath rate and tend to have high amplitudes. Again, these frequencies can be attenuated by the low-pass component of the band-pass filter in filter 130. Unlike leaks however, there is some overlap of frequency content of sighs/coughs and normal respiratory flows. Additional immunity to sighs and coughs can be gained if filter 130 compresses the signal as a function of instantaneous amplitude. This form of filter 130 represents just one possible method to tailor the behavior of controller 80. Any number of other processes are also possible. For example, adding a rectifier to filter 130 will cause the controller 80 to respond to one or the other half of the breathing cycle - namely the exhalation - as shown in FIG. 4 (i.e., "half-wave" mode). Without rectification in filter 130, the controller 80 will operate in "full-wave" mode as shown in FIG. 3. In this mode, the apparatus 10 can operate at generally lower pressures, enhancing patient comfort and, as a consequence, compliance.

Controller 80 and feedback path 120 can be implemented in hardware or software or a combination of both. One implementation of the present invention is software based using digital signal processing (DSP) methodologies. This method minimizes the cost to produce the apparatus and maximizes design flexibility. However, implementation of this invention is not construed to be fixed to DSP methods.

It is to be understood that other embodiments may be utilized and structural and functional changes may be made without departing from the scope of the present invention. The foregoing descriptions of embodiments of the invention have been presented for the purposes of illustration and description. It is not intended to be exhaustive or to limit the invention to the precise forms disclosed. For example, controller 80 may include additional components not specifically mentioned herein. Additionally, the feedback signal 120 may be taken from any point within controller 80 and is not limited to being taken from the output of controller 80. It is therefore intended that the scope of the invention be limited by the appended claims.

**Claims**

1. A positive airway pressure apparatus (10) comprising:

   a flow generator (30) coupled to a delivery device, the flow generator (30) producing a pressurized gas to the delivery device;
   a controller (80) having a control signal input (90) and configured to produce an output signal that drives the flow generator (30);
   a pressure sensor (50) configured to sense pressure in the positive airway pressure apparatus (10), the pressure sensor (50) providing a pressure sensor input signal (60) representing the pressure to the controller (80); and
   a feedback loop coupled to the controller (80) and providing a feedback signal (120) to the controller (80), the controller (80) including a summing component (70) to add three separate signals in order to provide an aggregate input to the controller (80), the three separate signals consisting of the control signal input (90), the feedback signal (120), and the pressure sensor input signal (60),
   wherein the summing component comprises three separate inputs corresponding to each of the three separate signals,
   wherein the feedback signal(120) is positive in polarity and the pressure signal (60) has a negative polarity,
   wherein the source of the feedback signal (120) is between an output of the summing component (70) and an input to the flow generator (30),
   wherein the controller (80) is configured to regulate the pressure by increasing the pressurized gas during inspiration and by decreasing the pressurized gas during expiration, thereby presenting a negative pressure impendance, and
   wherein the apparatus (80) does not comprise an additional flow sensing device.

**2.** The apparatus of claim 1, wherein the pressure sensor (50) senses pressure at the flow generator (30).

**3.** The apparatus (10) of claim 1, wherein the pressure sensor (50) senses at any point between the flow generator (30) and a patient interface (20).

**4.** The apparatus (10) of claim 1 further comprising a filter (130) as part of the feedback loop, wherein the filter (130) includes a high-pass component configured to remove frequencies below a lowest anticipated breath rate, and wherein the filter (130) includes a low pass component configured to remove frequencies above a highest anticipated breath rate.

**5.** The apparatus (10) of claim 4, wherein the filter (130) includes a rectifier, the rectifier causing the controller (80) to provide the pressurized gas to, only a portion of a breathing cycle.

**6.** The apparatus (10) of claim 5, wherein the portion of the breathing cycle is the inspiration cycle.

**7.** The apparatus (10) of claim 1, wherein the delivery device includes flexible tubing.

**8.** The apparatus (10) of claim 1, further comprising a patient interface (20) that allows the pressurized gas to be received by a patient.

**9.** The apparatus (10) of claim 8, wherein the patient interface (20) includes a mask.

**10.** The apparatus (10) of claim 1, wherein the apparatus (10) is a proportional positive airway pressure device.

**11.** The apparatus (10) of claim 1, wherein the apparatus (10) is used to treat sleep related breathing disorders and/or obstructive sleep apnea syndrome and/or chronic obstructive pulmonary disorder.

**12.** The apparatus (10) of claim 1, wherein the feedback loop is configured to provide the feedback input signal (120) from an output of the controller (80).


**Patentansprüche**

**1.** Positiv-Atemwegdruck-Gerät (10) aufweisend:

einen Strömungserzeuger (30), welcher mit einer Zuführvorrichtung verbunden ist, wobei der Strömungserzeuger (30) ein unter Druck stehendes Gas zu der Zuführvorrichtung produziert,
eine Steuerungseinheit (80), die einen Steuersignaleingang (90) hat und ausgebildet ist, ein Ausgangssignal zu produzieren, das den Strömungserzeuger (30) antreibt,
einen Drucksensor (50), der ausgebildet ist, um Druck in dem Positiv-Atemwegdruck-Gerät (10) abzufühlen, wobei der Drucksensor (50) ein Drucksensor-Eingangssignal (60) bereitstellt, das den Druck zu der Steuereinheit (80) repräsentiert, und
eine Rückführschleife, die mit der Steuereinheit (80) verbunden ist und ein Rückführsignal (120) an die Steuereinheit (80) bereitstellt, wobei die Steuereinheit (80) eine Summier-Komponente (70) zum Addieren von drei separaten Signalen aufweist, um einen Gesamteingang an die Steuereinheit (80) bereitzustellen, wobei die drei separaten Signale aus dem Steuersignaleingang (90), dem Rückführsignal (120) und dem Drucksensor-Eingangssignal (60) bestehen,
wobei die Summier-Komponente drei separate Eingänge aufweist, die mit jedem der drei separaten Signale korrespondieren,
wobei das Rückführsignal (120) in seiner Polarität positiv ist und das Drucksignal (60) eine negative Polarität hat, wobei die Quelle des Rückführsignals (120) zwischen einem Ausgang der Summier-Komponente (70) und einem Eingang zu dem Strömungserzeuger (30) ist,
wobei die Steuereinheit (80) ausgebildet ist, um den Druck durch Erhöhten des unter Druck stehenden Gases während der Inhalation und durch Verringern des unter Druck stehenden Gases während der Exhalation zu regulieren, wodurch eine Negativdruck-Impedanz präsentiert wird, und
wobei das Gerät (80) keine zusätzliche Strömungs-Abfühlvorrichtung aufweist.

**2.** Das Gerät gemäß Anspruch 1, wobei der Drucksensor (50) Druck an dem Strömungserzeuger (30) abfühlt.

**3.** Das Gerät (10) gemäß Anspruch 1, wobei der Drucksensor (50) an irgendeinem Punkt zwischen dem Strömungserzeuger (30) und einer Patienten-Schnittstelle (20) abführt.

**4.** Das Gerät (10) gemäß Anspruch 1, ferner aufweisen einen Filter (130) als Teil der Rückführschleife, wobei der Filter (130) eine Hochpasskomponente aufweist, um Frequenzen unterhalb einer untersten erwarteten Atmungsrate entfernt, und wobei der Filter (130) eine Tiefpasskomponente aufweist, die ausgebildet ist, um Frequenzen über einer höchsten erwarteten Atmungsrate zu entfernen.

**5.** Das Gerät (10) gemäß Anspruch 4, wobei der Filter (130) einen Gleichrichter aufweist, wobei der Gleichrichter die Steuereinheit (80) veranlasst, das unter Druck stehende Gas nur an einen Abschnitt eines Atmungskreislaufs bereitzustellen.

**6.** Das Gerät (10) gemäß Anspruch 5, wobei der Abschnitt des Atmungskreislaufs der Inhalationskreislauf ist.

**7.** Das Gerät (10) gemäß Anspruch 1, wobei die Zuführvorrichtung flexibles Schlauchmaterial aufweist.

**8.** Das Gerät (10) gemäß Anspruch 1, ferner aufweisend eine Patienten-Schnittstelle (20), die erlaubt, dass das unter Druck stehende Gas von dem Patienten erhalten wird.

**9.** Das Gerät (10) gemäß Anspruch 8, wobei die Patienten-Schnittstelle (20) eine Maske aufweist,

**10.** Das Gerät (10) gemäß Anspruch 1, wobei das Gerät (10) eine proportionale Positiv-Atemwegdruck-Vorrichtung ist.

**11.** Das Gerät (10) gemäß Anspruch 1, wobei das Gerät (10) zur Behandlung von schlafbezogenen Atmungsstörungen und/oder obstruktivem Schlafapnoe-Syndrom und/oder chronischer obstruktiver pulmonaler Störung verwendet wird.

**12.** Das Gerät (10) gemäß Anspruch 1, wobei die Rückführschleife ausgebildet ist, um das Rückführ-Eingangssignal (120) von einem Ausgang der Steuereinheit (80) bereitzustellen.

## Revendications

**1.** Appareil de pression des voies aériennes positive (10), comprenant :

un générateur de flux (30) couplé à un dispositif d'administration, le générateur de flux (30) produisant un gaz sous pression au niveau du dispositif d'administration ;
un contrôleur (80) présentant une entrée de signal de commande (90) et configuré de manière à produire un signal de sortie qui commande le générateur de flux (30) ;
un capteur de pression (50) configuré de manière à détecter une pression dans l'appareil de pression des voies aériennes positive (10), le capteur de pression (50) fournissant un signal d'entrée de capteur de pression (60) représentant la pression au contrôleur (80) ; et
une boucle de rétroaction couplée au contrôleur (80) et fournissant un signal de rétroaction (120) au contrôleur (80), le contrôleur (80) incluant un composant de sommation (70) destiné à additionner trois signaux distincts en vue de fournir une entrée agrégée au contrôleur (80), les trois signaux distincts étant constitués de l'entrée de signal de commande (90), du signal de rétroaction (120), et du signal d'entrée de capteur de pression (60) ;
dans lequel le composant de sommation comprend trois entrées distinctes correspondant à chacun des trois signaux distincts ;
dans lequel le signal de rétroaction (120) présente une polarité positive et le signal de pression (60) présente une polarité négative ;
dans lequel la source du signal de rétroaction (120) se situe entre une sortie du composant de sommation (70) et une entrée du générateur de flux (30) ;
dans lequel le contrôleur (80) est configuré de manière à réguler la pression en augmentant le gaz sous pression au cours de l'inspiration, et en diminuant le gaz sous pression au cours de l'expiration, ce qui permet de présenter une impédance de pression négative ; et
dans lequel l'appareil (80) ne comporte pas de dispositif supplémentaire de détection de flux.

**2.** Appareil selon la revendication 1, dans lequel le capteur de pression (50) détecte une pression au niveau du générateur de flux (30).

3. Appareil (10) selon la revendication 1, dans lequel le capteur de pression (50) détecte une pression au niveau d'un point quelconque entre le générateur de flux (30) et une interface de patient (20).

4. Appareil (10) selon la revendication 1, comprenant en outre un filtre (130) faisant partie de la boucle de rétroaction, dans lequel le filtre (130) comprend une composante passe-haut configurée de manière à supprimer des fréquences en dessous d'une fréquence respiratoire anticipée la plus faible, et dans lequel le filtre (130) comprend une composante passe-bas configurée de manière à supprimer des fréquences au-dessus d'une fréquence respiratoire anticipée la plus élevée.

5. Appareil (10) selon la revendication 4, dans lequel le filtre (130) inclut un redresseur, le redresseur amenant le contrôleur (80) à fournir le gaz sous pression à une partie seulement d'un cycle respiratoire.

6. Appareil (10) selon la revendication 5, dans lequel la partie du cycle respiratoire correspond au cycle d'inspiration.

7. Appareil (10) selon la revendication 1, dans lequel le dispositif d'administration inclut un tubage flexible.

8. Appareil (10) selon la revendication 1, comprenant en outre une interface de patient (20) qui permet la réception du gaz sous pression par un patient.

9. Appareil (10) selon la revendication 8, dans lequel l'interface de patient (20) inclut un masque.

10. Appareil (10) selon la revendication 1, dans lequel l'appareil (10) est un dispositif de pression des voies aériennes positive proportionnelle.

11. Appareil (10) selon la revendication 1, dans lequel l'appareil (10) est utilisé en vue de traiter des troubles respiratoires liés au sommeil et/ou un syndrome d'apnée du sommeil obstructive et/ou un trouble pulmonaire obstructif chronique.

12. Appareil (10) selon la revendication 1, dans lequel la boucle de rétroaction est configurée de manière à fournir le signal d'entrée de rétroaction (120) à partir d'une sortie du contrôleur (80).

FIG. 1

FLOW

0

INSPIRE

*FIG. 2*

PRESSURE

180

170

0

*FIG. 3*

PRESSURE

CPAP

0

TIME

*FIG. 4*

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9710019 A1